# EUROPÄISCHE PATENTSCHRIFT

(11) **EP 3 536 763 B1**
(45) Veröffentlichungstag und Bekanntmachung des Hinweises auf die Patenterteilung: **08.03.2023**
(21) Anmeldenummer: 19000123.0
(22) Anmeldetag: 08.03.2019
(51) Int. Cl.: C10G 9/00, F22B 1/02, F22B 1/18, F28C 3/08

(54) **QUENCHSYSTEM UND VERFAHREN ZUM KÜHLEN VON SPALTGAS EINES SPALTGASOFENS**
QUENCH SYSTEM AND PROCESS FOR COOLING A CRACKED GAS FROM A CRACKING FURNACE
SYSTÈME ET PROCÉDÉ DE REFROIDISSEMENT D'UN GAZ DE CRAQUAGE ISSU D'UN FOUR DE CRAQUAGE

(30) Priorität: 09.03.2018 DE 102018002086
(43) Veröffentlichungstag der Anmeldung: 11.09.2019
(73) Patentinhaber: Borsig GmbH, 13507 Berlin (DE)
(72) Erfinder: Birk, Carsten, 16548 Glienicke (DE); Drus, Sebastian, 15366 Hoppengarten (DE)
(74) Vertreter: Radünz, Ingo

(56) Entgegenhaltungen:
- EP-A1- 0 561 220
- EP-A1- 3 415 587
- DE-A1- 2 554 666
- DE-A1- 19 901 656
- DE-A1-102006 055 973
- US-A1- 2009 030 254
- US-A1- 2009 203 951
- US-A1- 2013 047 509

## Beschreibung

Die Erfindung bezieht sich auf ein Quenchsystem und ein Verfahren für ein Quenchsystem zum Kühlen von Spaltgas eines Spaltgasofens mit flüssigen als auch gasförmigen Ausgangsstoffen, welches einen Primary-Heat-Exchanger und einen Secondary-Heat-Exchanger und einen Tertiary-Heat-Exchanger umfasst, die in Reihe verbunden sind.

Für das Betreiben eines Ethylen-Ofens, der auch Spaltgasofen oder Cracking-Furnace genannt wird, gibt es verschiedene Ausgangsstoffe, die in einem Cracking-Furnace weiterverarbeitet werden. Solche Ausgangsstoffe sind unter anderem Naphtha, auch flüssiger Ausgangsstoff "Liquid-Feed" genannt, oder Gas, auch gasförmiger Ausgangsstoff "Gas-Feed" genannt, mit einem hohen Anteil an Ethylen. Beide Ausgangsstoffe werden in einem Cracking-Furnace hoch erwärmt und anschließend mit einem Quenchsystem, das auch kurz mit QS bezeichnet wird, schlagartig abgekühlt.

Je nach Ausgangsstoff wird ein speziell ausgelegtes Quenchsystem oder QS benötigt, weil die physikalischen Eigenschaften der Ausgangsstoffe unterschiedlich sind. Gas als Ausgangsstoff kann weiter herunter gekühlt werden, etwa von 900°C auf 150°C, als ein flüssiger Ausgangsstoff, der von etwa 900°C auf 350°C herunter gekühlt wird, weil die Kondensation von Gas erst bei deutlich geringeren Temperaturen einsetzt.

Demzufolge besteht ein Quenchsystem für Gas-Feed-Modus meistens aus einem Primary-Heat-Exchanger oder kurz PQE genannt und einem Secondary-Heat-Exchanger oder kurz SQE genannt und einem Tertiary-Heat-Exchanger oder auch kurz TQE genannt.

Ein Quenchsystem für Liquid-Feed-Modus besteht nur aus einem PQE und einem SQE, die in Reihe verbunden sind. Ein TQE, der immer als Speisewasservorwärmer oder Boiler-Feed-Water-Preheater dient, wird bei einer solchen Anordnung nicht installiert, wobei PQE und SQE jeweils als Verdampfer oder Evaporator geschaltet sind und als solche auch betrieben werden.

Als Beispiel einer möglichen Kühler-Anordnung für eine Betriebsweise eines Quenchsystems für Gas-Feed-Modus wird weiter unten auf eine nachfolgende Zeichnung verwiesen.

Als Beispiel einer möglichen Kühler-Anordnung für eine Betriebsweise eines Quenchsystems für Liquid-Feed-Modus wird ebenfalls weiter unten auf eine nachfolgende Zeichnung verwiesen.

Als ein Stand der Technik ist DE 10 2014 018 261 A1 zu nennen. Aus DE 10 2014 918 261 A1 ist ein Quenchkühlsystem mit einem primären Quenchkühler als Doppelrohrwärmetauscher und einem sekundären Quenchkühler als Rohrbündel-Wärmetauscher mit mindestens einem Rohrbündel bekannt, wobei das Rohrbündel von einem Mantel unter Bildung eines Mantelraumes umschlossen ist, der zwischen zwei mit Abstand voneinander angeordneten Rohrböden ausgebildet ist, zwischen denen jeweils Bündelrohre des Rohrbündels beidseitig in den Rohrböden gehalten sind.

Aus US 2009/0030254 A1 ist ein Verfahren und eine Vorrichtung zum Cracken von flüssigen Kohlenwasserstoffrückständen mit Dampf bekannt, wobei eine Trennvorrichtung für Dampf/Flüssigkeit benutzt wird, um aufgeheiztes Dampf-/Flüssigkeitsgemisch zu behandeln und für einen Dampfstrom mit reduziertem Rückstandsinhalt zu sorgen. Das Verfahren schließt ein: indirektes Wärmeaustauschen von flüssigen Destillationsrückständen mit Speisewasser oder Boiler-FeedWater, um flüssige Destillationsrückstände und vorgewärmtes Speisewasser zu liefern; Ableiten zumindest eines Teils des vorgewärmten Speisewassers an eine Dampftrommel und Rückgewinnen von Dampf mit einem Dampfdruck von mindestens etwa 4100 kPa aus der Dampftrommel. Die Vorrichtung umfasst unter anderem einen Liquid-Feed-Cracking-Furnace, eine Trennvorrichtung für Dampf/Flüssigkeit sowie einen primären Wärmetauscher, einen sekundären Wärmetauscher und einen zusätzlichen sekundären Wärmetauscher oder tertiären Wärmetauscher.

Weiter ist aus DE 10 2006 055 973 A1 ein Wärmetauscher zur Kühlung von Spaltgas in einer Ethylenanlage bekannt, bei dem vom Spaltgas durchströmte Wärmetauscherrohre an ihren jeweiligen Enden in jeweils eine Rohrplatte eingesetzt und von einem Mantel umgeben sind, an dessen beiden Stirnseiten je eine teilweise durch eine der Rohrplatten begrenzte Endkammer für die Zuführung und Abführung von Spaltgas vorgesehen ist. Der von dem Mantel umschlossene Innenraum des Wärmetauschers ist von Wasser als Kühlmedium durchströmt und durch eine senkrecht zu den Wärmetauscherrohren verlaufende und von den Wärmetauscherrohren durchdrungene Trennwand in zwei in Strömungsrichtung des Spaltgases hintereinander liegende Teilräume aufgeteilt, die mit jeweils eigenen Zuführungsstutzen und Abführungsstutzen für das Kühlmedium versehen sind.

Als weiteres Beispiel für ein bereits angewendetes Verfahren für Spaltgasöfen mit der Betriebsweise eines Quenchsystems für Gas-Feed-Modus und Liquid-Feed-Modus wird weiter unten auf eine nachfolgende Zeichnung hingewiesen.

Um ein Quenchsystem sowohl mit flüssigem als auch mit gasförmigem Ausgangsstoff betreiben zu können, muss ein PQE und ein SQE und ein TQE installiert sein. Bei einer Liquid-Feed-Betriebsweise ist der TQE gasseitig mittels Bypass zu umgehen.

Mit einer solchen Bypassschaltung wird die derzeitige Betriebsweise eines Quenchsystems für Gas-Feed als auch für Liquid-Feed nach dem Stand der Technik gemäß einer nachfolgenden Zeichnung durchgeführt. Bei einem Gas-Feed-Betrieb ist ein vor einem Gaseintrittsstutzen eines TQEs vorgesehenes Gaseintrittsventil offen und ein im Bypass des TQE angeordnetes Bypassventil geschlossen. Bei einem Liquid-Feed-Betrieb ist das Gaseintrittsventil geschlossen und das Bypassventil offen.

Nachteile der angewendeten Anordnung eines Quenchsystems für eine Betriebsweise Gas-Feed als auch Liquid-Feed nach dem Stand der Technik bestehen darin, dass eine solche Anordnung mit Bypasssteuerung sehr viel Platz benötigt und daher hohe Kosten verursacht, abgesehen davon, dass die technische Anordnung und Betriebsweise die an ein solches Quenchsystem gestellten Anforderungen hinsichtlich Zuverlässigkeit, Reparatur- und Wartungsfreundlichkeit nicht hinreichend erfüllen kann.

Die Aufgabe der Erfindung besteht darin, ein Quenchsystem und ein Verfahren für ein Quenchsystem zum Kühlen von Spaltgas eines Spaltgasofens mit flüssigen als auch gasförmigen Ausgangsstoffen zu schaffen, welches die hohen Anforderungen an technische Anordnung und Betriebsweise hinsichtlich Zuverlässigkeit und Kosten verbessert und eine einfache Möglichkeit hinsichtlich erforderlicher Reparatur- und Wartungsarbeiten gewährleistet.

Die vorliegende Aufgabe der Erfindung wird gattungsgemäß durch ein Quenchsystem gemäss Anspruch 1 und ein Verfahren gemäss Anspruch 9 gelöst, wobei ein Transfer Line Exchanger für einen Dual-Betrieb oder kurz TLX-D als ein Tertiary-Heat-Exchanger angeordnet und ausgebildet ist, dass der TLX-D mit einem Secondary-Heat-Exchanger oder SQE über eine TLX-D-Gaszuführungsleitung in Reihe geschaltet ist und dass der TLX-D über eine TLX-D-Speisewasserableitung mit darin angebrachtem TLX-D-Speisewasserableitungsventil und eine TLX-D-Steigleitung mit darin angeordnetem TLX-D-Steigleitungsventil und eine TLX-D-Fallleitung mit darin vorgesehenem TLX-D-Fallleitungsventil und der SQE über eine SQE-Fallleitung und eine SQE-Steigleitung mit einer Dampftrommel verbunden sind, welche an eine Speisewasserleitung angeschlossen ist, und dass der TLX-D eine TLX-D-Speisewasserzuleitung mit einem darin angebrachten TLX-D-Speisewasserzuleitungsventil aufweist, dass für TLX-D über die TLX-D-Fallleitung und die TLX-D-Steigleitung eine Kühlung das TLX-D im Naturumlauf vorgesehen ist und dass für TLX-D über die angeordnete TLX-D-Speisewasserzuleitung und die TLX-D-Speisewasserableitung eine Kühlung des TLX-D im Zwangsumlauf vorgesehen ist.

Der TLX-D ist für einen Dual-Betrieb so vorteilhaft angeordnet und ausgebildet, dass der TLX-D mit Abstand voneinander angeordnete Umlenkbleche aufweist, wobei die Umlenkbleche im vom TLX-D-Mantel umschlossenen TLX-D-Innenraum senkrecht zu einer Mittellinie des horizontal aufgestellten TLX-D angeordnet und Anordnung und Lage der Umlenkbleche aufgrund von für den Liquid-Feed-Modus mit zusätzlich entstehender Dampferzeugung vorbestimmt sind.

Ein weiterer Vorteil ist darin zu sehen, dass im TLX-D-Innenraum des TLX-D ein erstes Umlenkblech mit vorbestimmten Abstand zum TLX-D-Speisewassereintrittsstutzen angebracht ist und das erste Umlenkblech eine mantelseitige Speisewasserströmung um 180° umlenkt und einen freien Speisewasserströmungsquerschnitt aufweist, dessen maximale Höhe in Abhängigkeit von vorbestimmten Prozessbedingungen im Bereich von 10% bis 40%, vorzugsweise 15% bis 25% des TLX-D Mantelinnendurchmessers liegt. Weiter ist im TLX-D-Innenraum des TLX-D mit vorbestimmtem Abstand zum ersten Umlenkblech ein zweites Umlenkblech angeordnet, das Speisewasser um 180° umlenkt und einen freien Speisewasserströmungsquerschnitt aufweist. Eine weitere Anordnung von Umlenkblechen in Abhängigkeit von der Länge des TLX-D bis zum TLX-D-Speisewasseraustrittsstutzen ist vorgesehen.

Ein weiteres Merkmal liegt darin begründet, dass eine jeweilige Länge eines TLX-D mit vorgegebenen Prozessbedingungen auf der Gas- und Wasser-/Dampfseite vorbestimmt ist und dass eine Anzahl von angeordneten Umlenkblechen in Abhängigkeit von vorbestimmten Prozessbedingungen variabel ist, wobei der Abstand der jeweiligen Umlenkbleche voneinander in einem Bereich von ungefähr 100mm bis 800mm, vorzugsweise bei 300mm bis 600mm liegt.

Ein weiterer Vorteil besteht darin, dass beim TLX-D bei senkrecht zur TLX-D-Mittellinie durch den horizontal aufgestellten TLX-D strömendem Speisewasser die Umlenkbleche im oberen Bereich abgeflacht ausgeführt sind und dass unter den TLX-D-Steigleitungen ein freies Volumen oder ein Dampfraum ausgebildet ist.

Ein zusätzlicher Vorteil besteht darin, dass beim TLX-D die Abflachung der Umlenkbleche so klein gehalten ist, dass einerseits keine ungewollte Bypassströmung beim Betrieb des TLX-D als Speisewasservorwärmer im Gas-Feed-Modus entsteht, und so groß ausgebildet ist, dass andererseits der teilweise durch Phasenübergang des Wassers entstehende Dampfanteil beim Betrieb des TLX-D als Verdampfer im Liquid-Feed-Modus komplett abführbar ist. Bevorzugt ist, dass die maximale Höhe des Querschnitts der Abflachung in einem Bereich von ungefähr 5mm bis 40mm, vorzugsweise 10mm bis 15mm, ausgebildet ist.

Bei einem Verfahren gemäss Anspruch 9 für ein Quenchsystem zum Kühlen von Spaltgas eines Spaltgasofens mit flüssigen als auch gasförmigen Ausgangsstoffen hat sich als besonders vorteilhaft herausgestellt, dass ein als Tertiary-Heat-Exchanger ausgebildeter Transfer-Line-Exchanger für Dual-Betrieb oder TLX-D geschaltet wird und der TLX-D bei gasförmigem Ausgangsstoff im Gas-Feed-Modus als Speisewasservorwärmer und bei flüssigem Ausgangsstoff im Liquid-Feed-Modus als Verdampfer betrieben wird, wobei im Gas-Feed-Modus das TLX-D-Speisewasserzuleitungsventil und das TLX-D-Speisewasserableitungsventil geöffnet werden und das TLX-D-Fallleitungsventil und das TLX-D-Steigleitungsventil geschlossen werden, dass beim TLX-D im Liquid-Feed-Modus das TLX-D-Fallleitungsventil und das TLX-D-Steigleitungsventil geöffnet und das TLX-D-Speisewasserzuleitungsventil und das TLX-D-Speisewasserableitungsventil geschlossen werden und dass Speisewasser zur Dampftrommel über eine angebrachte Speisewasserzuleitung geführt wird.

Ein weiterer Vorteil bei dem Verfahren zeigt sich darin, dass im TLX-D über das geöffnete TLX-D-Speisewasserzuleitungsventil Speisewasser mantelseitig im Gegenstromprinzip entgegen der Strömungsrichtung des gasförmigen Spaltgases geführt wird, wobei das Speisewasser auf eine vorbestimmte Temperatur abgekühlt wird.

Ein weiterer Vorteil wird dadurch erzielt, dass im TLX-D durch abgeführte Wärme vom Spaltgas das geführte Speisewasser auf Temperaturen von etwa 150°C bis 300°C erwärmt wird. Vorteilig erweist sich auch, dass beim TLX-D im Liquid-Feed-Modus das TLX-D Fallleitungsventil und das TLX-D Steigleitungsventil geöffnet und das TLX-D Speisewasserzuleitungsventil und das TLX-D Speisewasserableitungsventil geschlossen werden und das Speisewasser zur Dampftrommel über eine angebrachte Speisewasserzuleitung geführt wird.

Ein weiterer Vorteil liegt darin, dass der TLX-D-Dual-Heat-Exchanger in das Sattdampfsystem oder Kühlsystem des Quenchsystems eingebunden wird, wobei Wasser aus der Dampftrommel über die TLX-D-Fallleitung und das geöffnete TLX-D-Fallleitungsventil bis zur Verteilung zu den am TLX-D angebrachten TLX-D-Fallleitungsstutzen geführt wird. Weiterhin ist vorteilhaft, dass beim TLX-D Wasser aus der Dampftrommel über die TLX-D-Fallleitung mantelseitig bis zu TLX-D-Steigleitungsstutzen, die den TLX-D-Fallleitungsstutzen gegenüberliegend angeordnet werden, geführt wird, wobei den TLX-D durchströmendes Spaltgas nicht wesentlich abgekühlt wird, nicht mehr als 15% Abkühlung der Spaltgaseintrittstemperatur, vorzugsweise weniger als 10%. Ein besonderer Vorteil ist darin zu sehen, dass beim TLX-D durch die spezielle Führung der Wasserströmung eine Spaltgaseintrittstemperatur nahe 50°C, vorzugsweise weniger als 30°C, oberhalb des Bereiches der Sattdampftemperatur liegt und auf der Wasserseite oder Mantelseite vom TLX-D-Mantel durch teilweisen Phasenübergang von Wasser eine geringe Menge entstehender Dampf, weniger als 10t/h Dampf, vorzugsweise weniger als 5t/h Dampf, erzeugt wird und der Dampf über die TLX-D-Steigleitungsstutzen, über TLX-D-Steigleitung, über geöffnetes TLX-D-Steigleitungsventil und TLX-D-Dampftrommelsteigleitungsstutzen in die Dampftrommel geleitet wird.

Als weiterer Vorteil hat sich herausgestellt, dass bei der Auslegung des freien Volumens oder des Dampfraums oder der Abflachung der Umlenkbleche berücksichtigt wird, dass die Abflachung der Umlenkbleche so klein bestimmt wird, dass einerseits keine ungewollte Bypassströmung beim Betrieb des TLX-D die Speisewasservorwärmer im Gas-Feed-Modus entstehen wird, und so groß ist, dass andererseits der durch teilweisen Phasenübergang des Wassers entstehende Dampfanteil beim Betrieb des TLX-D als Verdampfer im Liquid-Feed-Modus vollständig abgeführt wird, wobei die maximale Höhe des Querschnitts der Abflachung in einem Bereich von ungefähr 5mm bis 40mm, vorzugsweise 10mm bis 15mm, ausgebildet wird.

Der TLX-D ist für einen Dual-Betrieb so vorteilhaft angeordnet und ausgebildet, dass der TLX-D sowohl für eine Betriebsweise im Gas-Feed-Modus als auch im Liquid-Feed-Modus verwendbar ist und dass der TLX-D für einen solchen Dual-Betrieb sowohl als Speisewasservorwärmer als auch als Verdampfer vorgesehen ist.

Bei der vorteilhaften Anordnung des TLX-D erfolgt die Steuerung der Betriebsweise über den Wasser-/Dampfkreislauf und nicht mehr über die Gasseite der Gaszuführung vom PQE und SQE.

Weitere Einzelheiten und Vorteile der Erfindung sind anhand eines in der Zeichnung dargestellten Ausführungsbeispiels näher erläutert. Es zeigen:
Fig. 1 eine schematische Anordnung eines Kühlersystems für die Betriebsweise eines Quenchsystems für Gas-Feed nach dem Stand der Technik;
Fig. 2 eine schematische Anordnung eines Kühlersystems für die Betriebsweise eines Quenchsystems für Liquid-Feed nach dem Stand der Technik;
Fig. 3 eine schematische Anordnung eines Kühlersystems für die Betriebsweise eines Quenchsystems für Gas-Feed und Liquid-Feed mit Bypass nach dem Stand der Technik;
Fig. 4 ein bevorzugtes Ausführungsbeispiel einer Anordnung eines Kühlersystems für die Betriebsweise eines Quenchsystems für Gas-Feed und Liquid-Feed nach der vorliegenden Erfindung und
Fig. 5 ein bevorzugtes Ausführungsbeispiel einer Ausbildung eines Transfer-Line-Exchangers für die Betriebsweise eines Quenchsystems für Gas-Feed und Liquid-Feed nach der vorliegenden Erfindung.

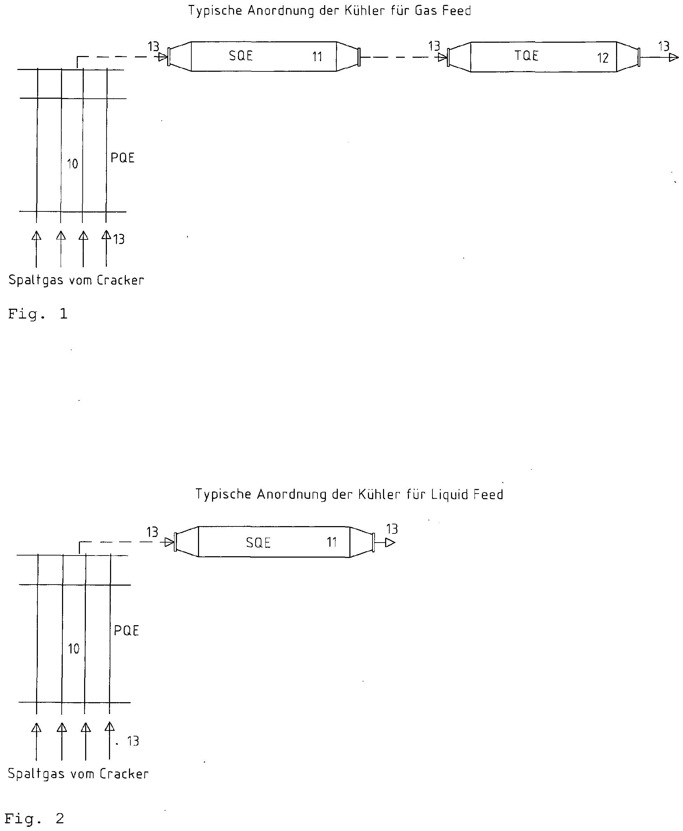

In Fig. 1 ist eine bekannte schematische Anordnung eines Kühlersystems für die Betriebsweise eines Quenchsystems für Gas-Feed-Modus gezeigt. Ein angedeuteter PQE 10 und ein SQE 11 und ein TQE 12 sind in Reihe geschaltet. Der PQE 10 und der SQE 11 sind als Verdampfer geschaltet, während der TQE 12 als Wasservorwärmer betrieben wird. Spaltgas oder Cracking Gas vom nicht dargestellten Cracking Furnace wird dem PQE 10, dem SQE 11 und dem TQE 12 in Richtung angedeuteter Pfeile 13 zugeführt.

In Fig. 2 ist eine bekannte schematische Anordnung eines Kühlersystems für die Betriebsweise eines Quenchsystems für Liquid-Feed-Modus gezeigt. Ein angedeuteter PQE 10 und ein SQE 11 sind in Reihe geschaltet und werden als Verdampfer betrieben. Ein TQE, der stets als Wasservorwärmer oder Boiler-Feed-Water-Preheater, auch kurz BFW-Preheater genannt, betrieben wird, ist nicht installiert. Spaltgas vom nicht dargestellten Cracking Furnace wird dem PQE 10 und dem SQE 11 in Richtung angedeuteter Pfeile 13 zugeführt.

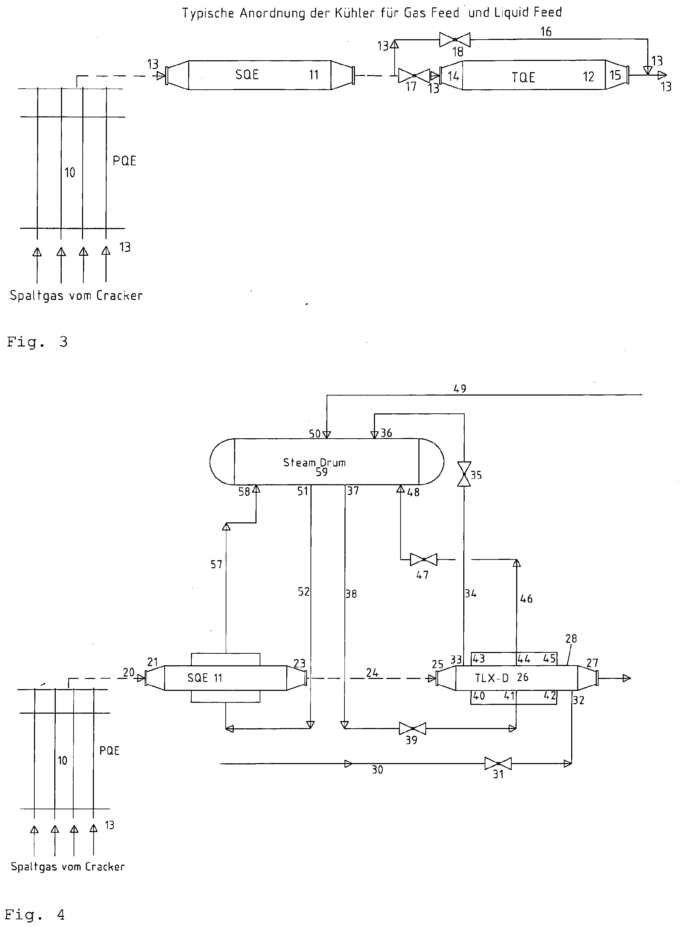

In Fig. 3 ist eine bekannte schematische Anordnung eines Kühlersystems für die Betriebsweise eines Quenchsystems für Gas-Feed-Modus und Liquid-Feed-Modus gezeigt. Ein angedeuteter PQE 10 und ein SQE 11 und ein TQE 12 sind in Reihe geschaltet. PQE 10 und SQE 11 werden als Verdampfer betrieben, während TQE 12 als Wasservorwärmer betrieben wird. Zwischen einem TQE-Gaseintrittsstutzen 14 und einem TQE-Gasaustrittsstutzen 15 in Pfeilrichtung des horizontal angeordneten TQEs 12 ist eine Bypassleitung 16 parallelgeschaltet. Die Bypassleitung 16 zweigt vor einem am TQE-Gaseintrittsstutzen 14 des TQEs 12 angeordneten TQE-Zuführventil 17 ab. In der Bypassleitung 16 ist ein TQE-Bypassventil 18 angeordnet. Spaltgas von einem nicht dargestellten Cracking Furnace wird dem PQE 10 und dem SQE 11 und dem TQE 12 in Richtung angedeuteter Pfeile 13 zugeführt.

In Fig. 4 ist ein bevorzugtes Ausführungsbeispiel einer Anordnung eines vorteilhaften Quenchsystems für Gas-Feed-Modus als auch Liquid-Feed-Modus schematisch gezeigt. Aus Gründen einer besseren Übersicht ist ein PQE nur schematisch dargestellt. Lediglich ist eine vom PQE 10 kommende und durch einen Pfeil angedeutete Leitung 20 gezeigt, die Spaltgas in Pfeilrichtung zu SQE-Gaseintrittsstutzen 21 eines Secondary-Heat-Exchangers oder kurz SQE 11 führt.

Der horizontal angeordnete SQE 11 ist spaltgasseitig in Reihe mit einem ebenfalls horizontal angeordneten Transfer-Line-Exchanger für dualen Betrieb geschaltet, der weiterhin kurz TLX-D 26 genannt ist. Ein zu kühlendes Spaltgas gelangt über die vorgesehene Leitung 20 in den SQE-Gaseintrittsstutzen 21 und durchströmt den SQE 11 bis zum SQE-Gasaustrittsstutzen 23. Über eine angeordnete TLX-D-Gaszuführungsleitung 24 strömt Spaltgas durch einen TLX-D-Gaseintrittsstutzen 25 bis zum TLX-D-Gasaustrittsstutzen 27 des TLX-D 26.

Der SQE 11 ist auf der Kühlseite oder Wasser-/Dampfseite oder Mantelseite über eine SQE-Fallleitung 52 und eine SQE-Steigleitung 57 mit einer Dampftrommel 59 verbunden. Über die SQE-Fallleitung 52 und die SQE-Steigleitung 57 erfolgt die Kühlung des SQE 11 im Naturumlauf.

Der TLX-D 26 ist weiterhin über eine angebrachte TLX-D-Fallleitung 38 einschließlich eines darin angeordneten TLX-D-Fallleitungsventils 39 und über eine angebrachte TLX-D-Steigleitung 46 einschließlich eines darin angeordneten TLX-D-Steigleitungsventils 47 mit der Dampftrommel 59 verbunden. Über die TLX-D-Fallleitung 38 und die TLX-D-Steigleitung 46 erfolgt die Kühlung des TLX-D 26 im Naturumlauf.

Weiterhin ist der TLX-D 26 an eine vorgesehene TLX-D-Speisewasserzuleitung 30 einschließlich eines darin angebrachten TLX-D-Speisewasserzuleitungsventils 31 angeschlossen und über eine vorgesehene TLX-D-Speisewasserableitung 34 einschließlich eines darin angebrachten TLX-D-Speisewasserableitungsventils 35 mit der Dampftrommel 59 verbunden. Über die angeordnete TLX-D-Speisewasserzuleitung 30 und die TLX-D-Speisewasserableitung 34 erfolgt eine Kühlung des TLX-D 26 im Zwangsumlauf.

Zur näheren Erläuterung der Funktionsweise des TLX-D 26 wird im Folgenden der SQE 11 nicht weiter betrachtet.

Der TLX-D 26 kann vorzugsweise in zwei verschiedenen Varianten betrieben werden. Abhängig von dem zu bearbeitenden Spaltgas wird bei gasförmigem Ausgangsstoff in der Betriebsweise Gas-Feed-Modus der TLX-D 26 als Speisewasservorwärmer und bei flüssigem Ausgangsstoff in der Betriebsweise Liquid-Feed-Modus als Verdampfer betrieben. Zu einer solchen unterschiedlichen Betriebsweise ist bereits einleitend eine nähere Erläuterung ausgeführt worden, so dass auf eine weitere Beschreibung verzichtet wird.

In der Betriebsweise Gas-Feed-Modus des TLX-D 26, der dann als Speisewasservorwärmer betrieben wird, sind das TLX-D-Speisewasserzuleitungsventil 31 und das TLX-D-Speisewasserableitungsventil 35 geöffnet und das TLX-D-Fallleitungsventil 39 und das TLX-D-Steigleitungsventil 47 geschlossen; d.h. die TLX-D-Fallleitung 38 und die TLX-D-Steigleitung 46 sind gesperrt und nicht mehr in Betrieb.

Eine Zulieferung von Speisewasser oder Boiler-Feed-Water-Supply erfolgt mittels einer nicht dargestellten Pumpe durch die TLX-D-Speisewasserzuleitung 30 über das offene TLX-D-Speisewasserzuleitungsventil 31 zum TLX-D-Speisewassereintrittsstutzen 32 des TLX-D 26. Speisewasser durchströmt auf die Weise den TLX-D 26 mantelseitig im Gegenstromprinzip, also entgegen der Strömungsrichtung des Spaltgases, bis zum TLX-D-Speisewasseraustrittsstutzen 33. Durch die besonders wirksame Strömungsführung des Speisewassers durch den TLX-D 26 im Gegenstromprinzip wird Spaltgas, das vom TLX-D-Gaseintrittsstutzen 25 durch den TLX-D 26 rohrseitig bis zum TLX-D-Gasaustrittsstutzen 27 strömt, in wirkungsvoller Weise auf eine vorbestimmte Temperatur abgekühlt. Durch das geführte Speisewasser wird die abgeführte Wärme aufgenommen, wobei das Speisewasser auf Temperaturen von etwa 150°C bis etwa 300°C erwärmt wird. Das erwärmte Speisewasser verlässt den TLX-D 26 über den angebrachten TLX-D-Speisewasseraustrittsstutzen 33 und wird durch die angeordnete TLX-D-Speisewasserableitung 34 und über das offene TLX-D-Speisewasserableitungsventil 35 durch einen an der Dampftrommel 59 angebrachten TLX-D-Dampftrommelspeisewasserstutzen 36 in die Dampftrommel 59 eingeleitet.

In der Betriebsweise Liquid-Feed-Modus des TLX-D 26, der dann als Verdampfer betrieben wird, sind das TLX-D-Fallleitungsventil 39 und das TLX-D-Steigleitungsventil 47 geöffnet und das TLX-D-Speisewasserzuleitungsventil 31 und das TLX-D-Speisewasserableitungsventil 35 geschlossen; d.h. die TLX-D-Speisewasserzuleitung 30 und die TLX-D-Speisewasserableitung 34 sind gesperrt und nicht in Betrieb. Eine Speisewasserzuführung zur Dampftrommel 59 erfolgt über eine angebrachte Speisewasserzuleitung 49 und einen an der Dampftrommel angeordneten Speisewasserstutzen 50. Das erforderliche Speisewasser wird der Dampftrommel 59 bei der Betriebsweise Liquid-Feed-Modus von einer externen Quelle zugeführt. Eine solche externe Lieferung von Speisewasser hat keinen Einfluss auf die Betriebsweise des TLX-D 26 und wird daher nicht weiter betrachtet.

Der TLX-D 26 ist in das Sattdampfsystem oder Kühlsystem des Quenchsystems eingebunden. Wasser aus der Dampftrommel 59 gelangt durch den TLX-D-Fallleitungsstutzen 37, über TLX-D-Fallleitung 38, über das offene TLX-D-Fallleitungsventil 39 bis zur Verteilung zu TLX-D-Fallleitungsstutzen 40, 41, 42, die am TLX-D 26 angebracht sind. Der TLX-D 26 wird vom Wasser mantelseitig von den TLX-D-Fallleitungsstutzen 40, 41, 42 bis zu den gegenüberliegenden TLX-D-Steigleitungsstutzen 43, 44, 45 durchströmt. Beim Durchströmen des TLX-D 26 wird Spaltgas, welches vom TLX-D-Gaseintrittsstutzen 25 durch TLX-D 26 rohrseitig bis zum TLX-D-Gasaustrittsstutzen 27 strömt, nicht signifikant, nicht mehr als 15% Abkühlung der Spaltgaseintrittstemperatur, vorzugsweise weniger als 10%, abgekühlt, da die Spaltgaseintrittstemperatur nahe 50°C, vorzugsweise weniger als 30°C, oberhalb des Bereiches der Sattdampftemperatur des Wassers liegt. Daher wird auf der Wasserseite oder Mantelseite des TLX-D 26 nur eine geringe Menge Dampf, weniger als 10 t/h Dampf, vorzugsweise 5 t/h Dampf, erzeugt, welcher durch die TLX-D-Steigleitungsstutzen 43, 44, 45, über TLX-D-Steigleitung 46 und über das offene TLX-D-Steigleitungsventil 47 und über TLX-D-Dampftrommelsteigleitungsstutzen 48 in die Dampftrommel 59 geleitet wird. Durch die bevorzugte Ausbildung kann der TLX-D 26 mit sehr geringer Leistungsabgabe betrieben werden. Durch eine solche Betriebsweise wird eine Abkühlung von Spaltgas unter die Kondensationstemperatur vermieden, ohne dass ein konventioneller TQE mit Hilfe von einem Bypass umgangen werden muss.

Die Vorteile bei dem bevorzugten Ausführungsbeispiel sind darin zu sehen, dass wesentliche Kosten dadurch gesenkt werden können, dass eine gasseitige Bypassschaltung vermieden werden kann und der damit verbundene aufwändige Platzbedarf entfallen kann.

Für einen Dual-Betrieb sind wesentliche technische Änderungen beim TLX-D 26 im Vergleich zu einem konventionellen TQE ausgebildet.

In dem Ausführungsbeispiel sind an dem TLX-D 26 in bevorzugter Weise der TLX-D-Speisewassereintrittsstutzen 32 und der TLX-D-Speiswasseraustrittsstutzen 33 für einen im Gas-Feed-Modus als Speisewasservorwärmer betriebenen TLX-D angeordnet. Außerdem sind jeweils vorzugsweise TLX-D-Fallleitungsstutzen 40, 41, 42 und TLX-D-Steigleitungsstutzen 43, 44, 45 für einen im Liquid-Feed-Modus als Verdampfer betriebenen TLX-D 26 angebracht.

Der TLX-D-Speisewassereintrittsstutzen 32 und der TLX-D-Speisewasseraustrittsstutzen 33 des horizontal angeordneten TLX-D 26 sind jeweils vor dem TLX-D-Gasaustrittsstutzen 27 bzw. hinter dem TLX-D-Gaseintrittsstutzen 25 jeweils auf der Unterseite bzw. der Oberseite am TLX-D Mantel 28 vorgesehen. Die Zuführung von Speisewasser erfolgt über den an der Unterseite des TLX-D-Mantels 28 angebrachten TLX-D-Speisewassereintrittsstutzen 32, und die Abführung des vorgewärmten Speisewassers erfolgt über den an der Oberseite des TLX-D-Mantels 28 angeordneten TLX-D-Speiswasseraustrittsstutzen 33.

Die Anzahl und Horizontallage der TLX-D-Fallleitungsstutzen 40, 41, 42 und der TLX-D-Steigleitungsstutzen 43, 44, 45 ist auf Basis der geforderten Dampferzeugung vorbestimmt; d.h. die in Fig. 4 dargestellte Anzahl der TLX-D-Steigleitungsstutzen und TLX-D-Fallleitungsstutzen ist variabel. Dabei erfolgt eine Wasserzuführung über die auf der Unterseite des TLX-D-Mantels 28 angebrachten TLX-D-Fallleitungsstutzen 40, 41, 42 und eine Wasser-/Dampfabführung über die auf der Oberseite des TLX-D-Mantels angeordneten TLX-D-Steigleitungsstutzen 43, 44, 45 des horizontal aufgestellten TLX-D 26.

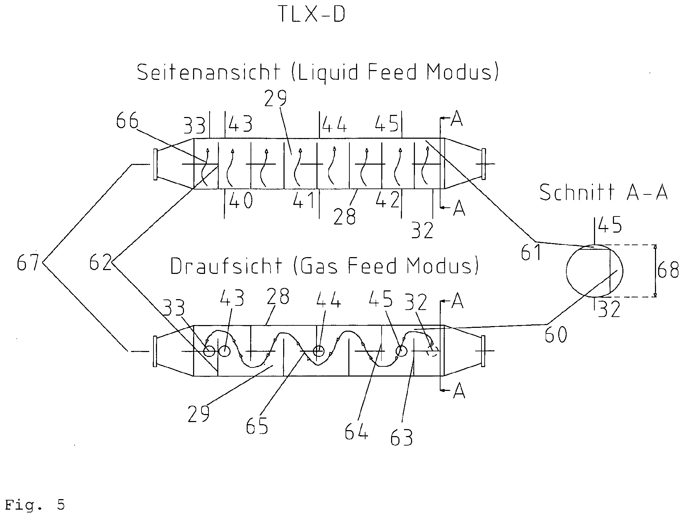

Eine Anordnung und Lage von Umlenkblechen 62 im TLX-D-Innenraum 29, der vom TLX-D-Mantel 28 des TLX-D 26 umschlossen wird, sind auf Grund der Spaltgasabkühlung im Gas-Feed-Modus vorbestimmt. Die Umlenkbleche 62 sind besonders ausgebildet, was weiter unten gezeigt und beschrieben ist. Eine solche Anordnung und Lage von Umlenkbleche 62 des TLX-D 26 sind in Fig. 5 dargestellt.

In einer Draufsicht von Fig.5 ist eine durch eine Schlangenlinie angedeutete mantelseitige Speisewasserströmung 65 im Gas-Feed-Modus dargestellt. Speisewasser tritt durch den TLX-D-Speisewassereintrittsstutzen 32 in den TLX-D 26 ein und wird durch ein im TLX-D-Innenraum 29 des TLX-D mit vorbestimmtem Abstand zum Speisewassereintrittsstutzen angebrachtes erstes Umlenkblech 63 um 180° umgelenkt und durchströmt dabei einen freien Speisewasserströmungsabschnitt 60, dessen Querschnitt aus Schnitt A-A ersichtlich ist und eine maximale Höhe in Abhängigkeit von vorbestimmten Prozessbedingungen im Bereich von 10% bis 40%, vorzugsweise 15% bis 25% des Durchmessers des TLX-D-Mantels 28 aufweist . Auch bei einem im TLX-D-Innenraum 29 des TLX-D 26 mit vorbestimmtem Abstand zum ersten Umlenkblech 63 angeordneten zweiten Umlenkblech 64 wird das Speisewasser um 180° umgelenkt und passiert einen zweiten freien Speisewasserströmungsabschnitt.

Ein solcher Vorgang wiederholt sich in Abhängigkeit von der Länge des TLX-D 26 bis zum TLX-D-Speisewasseraustrittsstutzen 33. Die jeweilige Länge eines TLX-D 26 ist mit vorgegebenen genauen Prozessbedingungen auf der Gas- und Wasser-/Dampfseite vorbestimmt. Die Anzahl der angeordneten Umlenkbleche 62 ist in Abhängigkeit von vorbestimmten Prozessbedingungen variabel. Der Abstand der jeweiligen Umlenkbleche voneinander liegt in einem Bereich von ungefähr 100mm bis 800mm, vorzugsweise jedoch 300mm bis 600mm.

In einer Seitenansicht von Fig.5 ist eine durch Pfeile angedeutete mantelseitige Wasser-/Dampfströmung 66 im Liquid-Feed-Modus dargestellt. Das Wasser tritt mantelseitig in den horizontal aufgestellten TLX-D 26 über die TLX-D-Fallleitungsstutzen 40, 41, 42 ein und durchquert den TLX-D senkrecht. Während das Wasser den TLX-D 26 senkrecht durchquert, findet ein teilweiser Phasenübergang des Wassers statt. Es liegt also neben Wasser auch ein Dampfanteil vor. Daher ist zu gewährleisten, dass entstehender Dampf über die TLX-D-Steigleitungsstutzen 43, 44, 45 abgeführt wird. Aus dem Grund sind die Umlenkbleche 62 im oberen Bereich abgeflacht ausgeführt. Dadurch entsteht unter den TLX-D-Steigleitungsstutzen 43, 44, 45 ein freies Volumen oder Dampfraum 61, in welchen entstehender Dampf strömt und über die TLX-D-Steigleitungsstutzen 43, 44, 45 abgeführt wird.

Bei der Auslegung des freien Volumens oder Dampfraums 61 oder der Abflachung der Umlenkbleche 62 ist zu berücksichtigen, dass die Abflachung der Umlenkbleche so klein ist, dass einerseits keine ungewollte Bypassströmung beim Betrieb des TLX-D 26 als Speisewasservorwärmer im Gas-Feed-Modus entsteht, und so groß ist, dass andererseits der entstehende Dampfanteil beim Betrieb des TLX-D als Verdampfer im Liquid-Feed-Modus komplett abführbar ist. Die maximale Höhe des Querschnitts der Abflachung soll in einem Bereich von ungefähr 5mm bis 40mm, vorzugsweise 10mm bis 15mm ausgebildet sein.

Eine Änderung der Anzahl und Lage von TLX-D-Fallleitungsstutzen und TLX-D-Steigleitungsstutzen sowie ein ausgebildetes Design von Umlenkblechen sind für eine sichere Betriebsweise eines TLX-D 26 im Dual-Betrieb ausschlaggebend. Daher sind die vorgegebenen Prozessbedingungen genau zu berücksichtigen, wenn ein TLX-D 26 ausgelegt wird.

## Patentansprüche

1. Quenchsystem für eine Anlage zum Kühlen von Spaltgas eines Spaltgasofens mit flüssigen als auch gasförmigen Ausgangsstoffen, welches einen Primary-Heat-Exchanger oder PQE (10) und einen Secondary-Heat-Exchanger oder SQE (11) und einen Tertiary-Heat-Exchanger oder TQE (12) umfasst, die in Reihe verbunden sind, **dadurch gekennzeichnet, dass** ein Transfer-Line-Exchanger für einen Dual-Betrieb oder TLX-D (26) als ein Tertiary-Heat-Exchanger angeordnet und ausgebildet ist, dass der TLX-D (26) mit Secondary-Heat-Exchanger SQE (11) über eine TLX-D-Gaszuführungsleitung (24) in Reihe geschaltet ist und dass der TLX-D (26) über eine TLX-D-Speisewasserableitung (34) mit darin angebrachtem TLX-D-Speisewasserableitungsventil (35) und eine TLX-D-Steigleitung (46) mit darin angeordnetem TLX-D-Steigleitungsventil (47) und eine TLX-D-Fallleitung (38) mit darin vorgesehenem TLX-D-Fallleitungsventil (39) und der SQE (11) über eine SQE-Fallleitung (52) und eine SQE-Steigleitung (57) mit einer Dampftrommel (59) verbunden sind, welche an eine Speisewasserleitung (49) angeschlossen ist, und dass der TLX-D (26) eine TLX-D-Speisewasserzuleitung (30) mit darin angebrachtem TLX-D-Speisewasserzuleitungsventil (31) aufweist, über die TLX-D-Fallleitung (38) und die TLX-D-Steigleitung (46) eine Kühlung des TLX-D (26) im Naturumlauf vorgesehen ist und dass für TLX-D (26) über die angeordnete TLX-D-Speisewasserzuleitung (30) und die TLX-D-Speisewasserableitung (34) eine Kühlung des TLX-D (26) im Zwangsumlauf vorgesehen ist.

2. Quenchsystem nach Patentanspruch 1, **dadurch gekennzeichnet, dass** der TLX-D (26) mit Abstand voneinander angeordnete Umlenkbleche (62) aufweist, dass die Umlenkbleche (62) im vom TLX-D-Mantel (28) umschlossenen TLX-D-Innenraum (29) senkrecht zu einer TLX-D-Mittellinie (67) des horizontal aufgestellten TLX-D (26) angeordnet sind, wobei Anordnung und Lage der Umlenkbleche (62) aufgrund von beim Liquid-Feed-Modus mit zusätzlich entstehender Dampferzeugung vorbestimmt sind.

3. Quenchsystem nach Patentanspruch 2, **dadurch gekennzeichnet, dass** im TLX-D-Innenraum (29) des TLX-D (26) ein erstes Umlenkblech (63) mit vorbestimmtem Abstand zum TLX-D-Speisewassereintrittsstutzen (32) angebracht ist, dass das erste Umlenkblech (63) eine mantelseitige Speisewasserströmung um 180° umlenkt und einen freien Speisewasserströmungsquerschnitt (60) aufweist, dessen maximale Höhe in Abhängigkeit von vorbestimmten Prozessbedingungen im Bereich von 10% bis 40%, vorzugsweise 15% bis 25% des TLX-D Mantelinnendurchmessers (68) liegt, dass im TLX-D-Innenraum (29) des TLX-D (26) mit vorbestimmtem Abstand zum ersten Umlenkblech (63) ein zweites Umlenkblech (64) angeordnet ist, das Speisewasser um 180° umlenkt und einen freien Speisewasserströmungsquerschnitt (60) aufweist, und dass eine weitere Anordnung von Umlenkblechen in Abhängigkeit von der Länge des TLX-D (26) bis zum TLX-D-Speisewasseraustrittsstutzen (33) vorgesehen ist.

4. Quenchsystem nach Patentanspruch 3, **dadurch gekennzeichnet, dass** eine jeweilige Länge eines TLX-D (26) mit vorgegebenen Prozessbedingungen auf der Gas- und Wasser-/Dampfseite vorbestimmt ist und dass eine Anzahl von angeordneten Umlenkblechen (62) in Abhängigkeit von vorbestimmten Prozessbedingungen variabel ist, wobei der Abstand der jeweiligen Umlenkbleche voneinander in einem Bereich von ungefähr 100mm bis 800mm oder eingegrenzt bei 300mm bis 600mm liegt.

5. Quenchsystem nach Patentanspruch 2, **dadurch gekennzeichnet, dass** beim TLX-D (26) die bei senkrecht zur TLX-D-Mittellinie (67) durch den horizontal aufgestellten TLX-D strömendem Speisewasser angeordneten Umlenkbleche (62) im oberen Bereich abgeflacht ausgeführt sind und dass unter den TLX-D-Steigleitungen (43, 44, 45) ein freies Volumen oder ein Dampfraum (61) ausgebildet ist.

6. Quenchsystem nach Patentanspruch 5, **dadurch gekennzeichnet, dass** die Abflachung der Umlenkbleche (62) so klein gehalten ist, dass einerseits keine ungewollte Bypassströmung beim Betrieb des TLX-D (26) als Speisewasservorwärmer im Gas-Feed-Modus entsteht, und so groß ausgebildet ist, dass andererseits der durch teilweisen Phasenübergang des Wassers entstehende Dampfanteil beim Betrieb des TLX-D (26) als Verdampfer im Liquid-Feed-Modus komplett abführbar ist, und dass die maximale Höhe des Querschnitts der Abflachung in einem Bereich von ungefähr 5mm bis 40mm , vorzugsweise 10mm bis 15mm, ausgebildet ist.

7. Verfahren für ein Quenchsystem zum Kühlen von Spaltgasen eines Spaltgasofens mit flüssigen als auch gasförmigen Ausgangsstoffen, welches einen PQE (10) und einen SQE (11) und einen TQE (12) umfasst, nach Patentanspruch 1, **dadurch gekennzeichnet, dass** ein als Tertiary-Heat-Exchanger ausgebildeter Transfer-Line-Exchanger für Dual-Betrieb oder TLX-D (26) geschaltet wird, dass der TLX-D (26) bei gasförmigem Ausgangsstoff im Gas-Feed-Modus als Speisewasservorwärmer und bei flüssigem Ausgangsstoff im Liquid-Feed-Modus als Verdampfer betrieben wird, dass im Gas-Feed-Modus das TLX-D-Speisewasserzuleitungsventil (31) und das TLX-D-Speisewasserableitungsventil (35) geöffnet werden und das TLX-D-Fallleitungsventil (39) und das TLX-D-Steigleitungsventil (47) geschlossen werden, dass beim TLX-D (26) im Liquid-Feed-Modus das TLX-D-Fallleitungsventil (39) und das TLX-D-Steigleitungsventil (47) geöffnet und das TLX-D-Speisewasserzuleitungsventil (31) und das TLX-D-Speisewasserableitungsventil (35) geschlossen werden und dass Speisewasser zur Dampftrommel (59) über eine angebrachte Speisewasserzuleitung (49) geführt wird.

8. Verfahren für ein Quenchsystem nach Patentanspruch 7, **dadurch gekennzeichnet, dass** im TLX-D (26) über das geöffnete TLX-D-Speisewasserzuleitungsventil (31) Speisewasser mantelseitig im Gegenstromprinzip entgegen der Strömungsrichtung des gasförmigen Spaltgases geführt wird, wobei das Speisewasser auf eine vorbestimmte Temperatur abgekühlt wird.

9. Verfahren für ein Quenchsystem nach Patentanspruch 8, **dadurch gekennzeichnet, dass** im TLX-D (26) über das geöffnete TLX-D-Speisewasserzuleitungsventil (31) Speisewasser mantelseitig im Gegenstromprinzip entgegen der Strömungsrichtung des gasförmigen Spaltgases geführt wird, wobei durch abgeführte Wärme von Spaltgas das geführte Speisewasser auf Temperaturen von etwa 150°C bis etwa 300°C erwärmt wird.

10. Verfahren für ein Quenchsystem nach Patentanspruch 7, **dadurch gekennzeichnet, dass** beim TLX-D (26) im Liquid-Feed-Modus das Speisewasser zur Dampftrommel (59) über die angebrachte Speisewasserzuleitung (49) und einen an der Dampftrommel angeordneten Speisewasserstutzen (50) geführt wird.

11. Verfahren für ein Quenchsystem nach Patentanspruch 7, **dadurch gekennzeichnet, dass** der TLX-D-Dual-Heat-Exchanger oder TLX-D (26) in das Sattdampfsystem oder Kühlsystem des Quenchsystems eingebunden wird, wobei Wasser aus der Dampftrommel (59) über die TLX-D-Fallleitung (38) und das geöffnete TLX-D-Fallleitungsventil (39) bis zur Verteilung zu den am TLX-D angebrachten TLX-D-Fallleitungsstutzen (40, 41, 42) geführt wird.

12. Verfahren für ein Quenchsystem nach Patentanspruch 11, **dadurch gekennzeichnet, dass** beim TLX-D (26) Wasser aus der Dampftrommel (59) über die TLX-D-Fallleitung (38) mantelseitig bis zu TLX-D-Steigleitungsstutzen (43, 44, 45), die den TLX-D-Fallleitungsstutzen (40, 41, 42) gegenüberliegend angeordnet werden, geführt wird, wobei den TLX-D (26) durchströmendes Spaltgas nicht wesentlich abgekühlt wird, nicht mehr als 15% Abkühlung der Spaltgaseintrittstemperatur, vorzugsweise weniger als 10%.

13. Verfahren für ein Quenchsystem nach Patentanspruch 12, **dadurch gekennzeichnet, dass** beim TLX-D (26) die Spaltgaseintrittstemperatur nahe 50°C, vorzugsweise weniger als 30°C, oberhalb des Bereiches der Sattdampftemperatur liegt, dass auf der Wasserseite oder Mantelseite vom TLX-D-Mantel (28) durch teilweisen Phasenübergang des Wassers eine geringe Menge entstehender Dampf, weniger als 10 t/h Dampf, vorzugsweise weniger als 5 t/h Dampf, über die TLX-D-Steigleitungsstutzen (43, 44, 45), über TLX-D-Steigleitung (46), über geöffnetes TLX-D-Steigleitungsventil (47) und TLX-D-Dampftrommelsteigleitungsstutzen (48) in die Dampftrommel (59) geleitet wird.

14. Verfahren für ein Quenchsystem nach Patentanspruch 13, **dadurch gekennzeichnet, dass** bei der Auslegung des freien Volumens oder des Dampfraums (61) oder der Abflachung der Umlenkbleche (62) berücksichtigt wird, dass die Abflachung der Umlenkbleche (62) so klein bestimmt wird, dass einerseits keine ungewollte Bypassströmung beim Betrieb des TLX-D (26) als Speisewasservorwärmer im Gas-Feed-Modus entstehen wird, und so groß ist, dass andererseits der durch teilweisen Phasenübergang des Wassers entstehende Dampfanteil beim Betrieb des TLX-D (26) als Verdampfer im Liquid-Feed-Modus vollständig abgeführt wird, wobei die maximale Höhe des Querschnitts der Abflachung in einem Bereich von ungefähr 5mm bis 40mm, vorzugsweise 10mm bis 15mm, ausgebildet wird.

## Claims

1. A quenching system for a plant for cooling cracked gas of a cracking furnace with liquid as well as gaseous starting materials, comprising a primary heat exchanger or PQE (10) and a secondary heat exchanger or SQE (11) and a tertiary heat exchanger or TQE (12) which are connected in series, **characterized in that** a transfer-line-exchanger for dual operation or TLX-D (26) is arranged and configured as a tertiary heat exchanger, that the TLX-D (26) is connected in series with the secondary heat exchanger SQE (11) via a TLX-D gas feed line (24), and that the TLX-D (26), via a TLX-D feed water drain line (34) with a TLX-D feed water drain line valve (35) installed therein and a TLX-D riser (46) with a TLX-D riser valve (47) arranged therein and a TLX-D downcomer (38) with a TLX-D downcomer valve (39) provided therein, and the SQE (11), via an SQE downcomer (52) and an SQE riser (57), are connected to a steam drum (59) which is connected to a feed water line (49), and that the TLX-D (26) has a TLX-D feed water supply line (30) with a TLX-D feed water supply line valve (31) installed therein, a cooling of the TLX-D (26) with natural circulation is provided via the TLX-D downcomer (38) and the TLX-D riser (46), and that a cooling of the TLX-D (26) with forced circulation is provided for the TLX-D (26) via the arranged TLX-D feed water supply line (30) and the TLX-D feed water drain line (34).

2. The quenching system according to claim 1, **characterized in that** the TLX-D (26) has baffles (62) arranged at a distance from one another, that the baffles (62) are arranged, in the TLX-D interior (29) enclosed by the TLX-D jacket (28), perpendicular to a TLX-D center line (67) of the horizontally positioned TLX-D (26), wherein the arrangement and position of the baffles (62) are predefined based on steam generation additionally occurring in the liquid feed mode.

3. The quenching system according to claim 2, **characterized in that** a first baffle (63) is installed in the TLX-D interior (29) of the TLX-D (26) at a predefined distance to the TLX-D feed water inlet pipe (32), that the first baffle (63) deflects a jacket-side feed water flow by 180° and has a free feed water flow cross section (60), the maximum height of which, as a function of predefined process conditions, is in the range of 10% to 40%, preferably 15% to 25%, of the TLX-D jacket internal diameter (68), that a second baffle (64) is arranged in the TLX-D interior (29) of the TLX-D (26) at a predefined distance to the first baffle (63), which deflects the feed water by 180° and has a free feed water flow cross section (60), and that a further arrangement of baffles is provided as a function of the length of the TLX-D (26) up to the TLX-D feed water outlet pipe (33).

4. The quenching system according to claim 3, **characterized in that** a respective length of a TLX-D (26) with predefined process conditions on the gas and water/steam side is predefined and that a number of arranged baffles (62) is variable as a function of predefined process conditions, wherein the distance of the respective baffles from another is in a range of about 100mm to 800mm or limited to about 300mm to 600mm.

5. The quenching system according to claim 2, **characterized in that** in the TLX-D (26), the baffles (62) arranged perpendicular to the TLX-D center line (67) when feed water flows through the horizontally positioned TLX-D are configured to be flattened in the upper region, and that a free volume or a steam space (61) is formed below the TLX-D riser pipes (43, 44, 45).

6. The quenching system according to claim 5, **characterized in that** the flattening of the baffle (62) is kept small enough that, on the one hand, no undesired bypass flow occurs in the gas feed mode during operation of the TLX-D (26) as a feed water preheater, and is configured large enough that, on the other hand, the steam content resulting from partial phase transition of the water can be completely discharged in the liquid feed mode during the operation of the TLX-D (26) as an evaporator, and that the maximum height of the cross section of the flattening is configured to be in a range of about 5mm to 40mm, preferably 10mm to 15mm.

7. A method for a quenching system for cooling cracked gases of a cracking furnace with liquid as well as gaseous starting materials, which comprises a PQE (10) and a SQE (11) and a TQE (12), according to claim 1, **characterized in that** a transfer-line-exchanger configured as a tertiary heat exchanger is connected for dual operation or TLX-D (26), that the TLX-D (26) is operated in gas feed mode as a feed water preheater in the case of gaseous starting material, that the TLX-D feed water supply valve (31) and the TLX-D feed water drain valve (35) are opened and the TLX-D downcomer valve (39) and the TLX-D riser valve (47) are closed in the gas feed mode, that in the TLX-D (26), the TLX-D downcomer valve (39) and the TLX-D riser valve (47) are opened and the TLX-D feed water supply valve (31) and the TLX-D feed water drain valve, (35) are closed in liquid feed mode, and that feed water is guided to the steam drum (59) via an installed feed water supply line (49).

8. The method for a quenching system according to claim 7, **characterized in that** in the TLX-D (26), feed water is guided against the flow direction of the gaseous cracked gas on the jacket side according to the counterflow principle via the open TLX-D feed water feed valve (31), wherein the feed water is cooled to a predefined temperature.

9. The method for a quenching system according to claim 8, **characterized in that** in the TLX-D (26), feed water is guided against the flow direction of the gaseous cracked gas on the jacket side according to the counterflow principle via the open TLX-D feed water supply valve (31), wherein the guided feed water is heated to temperatures of about 150°C to about 300°C by heat discharged from cracked gas.

10. The method for a quenching system according to claim 7, **characterized in that** in liquid feed mode in the TLX-D (26), the feed water is guided to the steam drum (59) via the installed feed water supply line (49) and a feed water pipe (50) arranged on the steam drum.

11. The method for a quenching system according to claim 7, **characterized in that** the TLX-D dual heat exchanger or TLX-D (26) is integrated into the saturated steam system or cooling system of the quenching system, wherein water from the steam drum (59) is guided via the TLX-D downcomer (38) and the opened TLX-D downcomer valve (39) to distribution to the TLX-D downcomer pipes installed at the TLX-D (40, 41, 42).

12. The method for a quenching system according to claim 11, **characterized in that** in the TLX-D (26), water is guided from the steam drum (59) via the TLX-D downcomer (38) on the jacket side up to TLX-D riser pipes (43, 44, 45), which are arranged opposite the TLX-D downcomer pipes (40, 41, 42), wherein cracked gas flowing through the TLX-D (26) is not significantly cooled, not more than 15% cooling of the cracked gas inlet temperature, preferably less than 10%.

13. The Method for a quenching system according to claim 12, **characterized in that** in the TLX-D (26), the cracked gas inlet temperature is close to 50°C, preferably less than 30°C, above the range of the saturated steam temperature, that on the water side or jacket side, a small amount of generated steam, less than 10 t/h steam, preferably less than 5 t/h steam, is guided from the TLX-D jacket (28) by partial phase transition of the water into the steam drum (59) via the TLX-D riser pipes (43, 44, 45), via TLX-D riser (46), via open TLX-D riser valve (47) and TLX-D steam drum riser pipe (48).

14. The method for a quenching system according to claim 13, **characterized in that**, when configuring the free volume or the steam space (61) or the flattening of the baffles (62), it is taken into account that the flattening of the baffles (62) is defined to be so small that, on the one hand, no unwanted bypass flow will occur in the gas feed mode during the operation of the TLX-D (26) as a feedwater preheater, and so large that, on the other hand, the steam content resulting from partial phase transition of the water is completely discharged in liquid feed mode during operation of the TLX-D (26) as an evaporator, wherein the maximum height of the cross section of the flattening is configured to be in a range of about 5mm to 40mm, preferably 10mm to 15mm.

## Revendications

1. Système de trempe, destiné à une installation de refroidissement de gaz de craquage d'un four de craquage à gaz avec des substances de départ aussi bien liquides que gazeuses, lequel comprend un Primary-Heat-Exchanger ou PQE (échangeur thermique primaire) (10) et un Secondary-Heat-Exchanger ou SQE (échangeur thermique secondaire)(11) et un Tertiary-Heat-Exchanger ou TQE (échangeur thermique tertiaire) (12) qui sont connectés en série, **caractérisé en ce qu'**un Transfer-Line-Exchanger (échangeur thermique sur ligne de transition) pour un mode dual ou TLX-D (26) est placé et conçu en tant que Tertiary-Heat-Exchanger, **en ce que** le TLX-D (26) est branché en série avec le Secondary-Heat-Exchanger SQE (11) par l'intermédiaire d'une conduite d'alimentation de gaz du TLX-D (24) et **en ce que** le TLX-D (26) est connecté par l'intermédiaire d'une évacuation d'eau d'alimentation du TLX-D (34) pourvue d'une soupape d'évacuation d'eau d'alimentation du TLX-D (35) montée en son intérieur et d'une conduite ascendante du TLX-D (46) pourvue d'une soupape de conduite ascendante du TLX-D (47) placée en son intérieur et d'une conduite descendante du TLX-D (38) pourvue d'une soupape de conduite descendante du TLX-D (39) prévue en son intérieur et **en ce que** le SQE (11) est connecté par l'intermédiaire d'une conduite descendante du SQE (52) et d'une conduite ascendante du SQE (57) avec un ballon à vapeur (59), lequel est raccordé sur une conduite d'eau d'alimentation (49) et **en ce que** le TLX-D (26) comporte une conduite d'arrivée d'eau d'alimentation du TLX-D (30) pourvue d'une soupape de conduite d'arrivée d'eau d'alimentation du TLX-D (31) montée en son intérieur, **en ce que** par l'intermédiaire de la conduite descendante de TLX-D (38) et de la conduite ascendante du TLX-D (46), il est prévu un refroidissement du TLX-D (26) en circulation naturelle et **en ce que** pour le TLX-D (26), un refroidissement du TLX-D (26) en circulation forcée est prévu par l'intermédiaire de la conduite d'arrivée d'eau d'alimentation (30) du TLX-D placée et de l'évacuation d'eau d'alimentation du TLX-D (34).

2. Système de trempe selon la revendication 1 du brevet, **caractérisé en ce que** le TLX-D (26) comporte des déflecteurs (62) placés avec un écart mutuel, **en ce que** les déflecteurs (62) sont placés dans l'espace intérieur du TLX-D (29) entouré par l'enveloppe du TLX-D (28) à la perpendiculaire d'une ligne médiane de TLX-D (67) du TLX-D (26) implanté à l'horizontale, le placement et la position des déflecteurs (62) étant prédéterminés du fait d'une génération de vapeur supplémentaire dans le mode d'alimentation de liquide.

3. Système de trempe selon la revendication 2 du brevet, **caractérisé en ce que** dans l'espace intérieur de TLX-D (29) du TLX-D (26) un premier déflecteur (63) est monté avec un écart prédéfini par rapport à la tubulure d'entrée d'eau d'alimentation de TLX-D (32), **en ce que** le premier déflecteur (63) dévie de 180° une circulation d'eau du côté de l'enveloppe et comporte une section transversale libre d'écoulement (60) d'eau d'alimentation dont la hauteur maximale se situe en fonction de conditions prédéfinies du processus dans l'ordre de 10 % à 40 %, de préférence de 15 % à 25 % du diamètre intérieur de l'enveloppe (68) du TLX-D, **en ce que** dans l'espace intérieur (29) de TLX-D du TLX-D (26), un deuxième déflecteur (64) est placé à un écart prédéfini du premier déflecteur (63), dévie l'eau d'alimentation de 180° et comporte une section transversale libre d'écoulement (60) d'eau d'alimentation, et **en ce qu'**un autre ensemble de déflecteurs est prévu en fonction de la longueur du TLX-D (26) jusqu'à la tubulure de sortie d'eau d'alimentation (33) du TLX-D.

4. Système de trempe selon la revendication 3 du brevet, **caractérisé en ce qu'**une longueur respective d'un TLX-D (26) est prédéfinie avec des conditions de processus prédéterminées sur le côté gaz/vapeur et eau/vapeur et **en ce qu'**un nombre de déflecteurs (62) placés est variable en fonction de conditions de processus prédéterminées, l'écart mutuel entre les déflecteurs respectifs se situant dans un ordre d'environ 100 mm à 800 mm ou étant limité à entre 300 mm et 600 mm.

5. Système de trempe selon la revendication 2 du brevet, **caractérisé en ce que** sur le TLX-D (26), les déflecteurs (62) placés dans l'eau d'alimentation circulant à travers le TLX-D implanté à l'horizontale à la perpendiculaire de la ligne médiane du TLX-D (67) sont réalisés avec un méplat dans la zone supérieure et **en ce qu'**en-dessous des conduites ascendantes (43, 44, 45) du TLX-D est conçu un volume libre ou un espace de vapeur (61).

6. Système de trempe selon la revendication 5 du brevet, **caractérisé en ce que** le méplat des déflecteurs (62) est maintenu à une dimension si faible, que d'une part il ne soit donné naissance à aucun courant de dérivation intempestif lors du fonctionnement du TLX-D (26) en tant que préchauffeur de l'eau de dérivation en mode d'alimentation de gaz, et est conçu à une dimension si importante, que d'autre part, lors du fonctionnement du TLX-D (26) en tant qu'évaporateur, la fraction de vapeur provoquée par transition partielle de phase de l'eau en mode d'alimentation de liquide soit complètement évacuable, et **en ce que** la hauteur maximale de la section transversale du méplat se situe dans un ordre d'environ 5 mm à 40 mm , de préférence de 10 mm à 15 mm.

7. Procédé destiné à un système de trempe pour refroidir des gaz de craquage d'un four de craquage à gaz avec des substances de départ aussi bien liquides que gazeuses, lequel comprend un PQE (10) et un SQE (11) et un TQE (12) selon la revendication 1 du brevet, **caractérisé en ce que** l'on branche un échangeur thermique sur ligne de transition pour un mode dual ou TLX-D (26) conçu sous la forme d'un échangeur thermique tertiaire, pour un mode dual ou TLX-D (26), **en ce qu'**avec une substance de départ gazeuse, en mode d'alimentation de gaz, le TLX-D (26) fonctionne en tant que préchauffeur d'eau d'alimentation et avec une substance de départ liquide, en mode d'alimentation de liquide, fonctionne en tant qu'évaporateur, **en ce que** dans le mode d'alimentation de gaz, l'on ouvre la soupape de conduite d'arrivée d'eau d'alimentation (31) du TLX-D et la soupape d'évacuation d'eau d'alimentation (35) du TLX-D et l'on ferme la soupape de conduite descendante (39) du TLX-D et la soupape de conduite ascendante (47) du TLX-D, (26) **en ce qu'**en mode d'alimentation de liquide, l'on ouvre sur le TLX-D la soupape de conduite descendante (39) du TLX-D et la soupape de conduite ascendante (47) du TLX-D et l'on ferme la soupape de conduite d'arrivée d'eau d'alimentation (31) du TLX-D et la soupape d'évacuation d'eau d'alimentation (35) du TLX-D et l'on conduit l'eau d'alimentation vers le ballon à vapeur (59) par l'intermédiaire d'une conduite d'arrivée d'eau d'alimentation (49) adaptée.

8. Procédé destiné à un système de trempe selon la revendication 7 du brevet, **caractérisé en ce que** dans le TLX-D (26), l'on conduit l'eau d'alimentation par l'intermédiaire de la soupape de conduite d'arrivée d'eau d'alimentation (31) ouverte du TLX-D, du côté de l'enveloppe, selon le principe à contre-courant à l'encontre de la direction d'écoulement du gaz de craquage gazeux, l'eau d'alimentation étant refroidie à une température prédéfinie.

9. Procédé destiné à un système de trempe selon la revendication 8 du brevet, **caractérisé en ce que** dans le TLX-D (26), l'on conduit l'eau d'alimentation par l'intermédiaire de la soupape de conduite d'arrivée d'eau d'alimentation (31) ouverte du TLX-D, du côté de l'enveloppe selon le principe à contre-courant à l'encontre de la direction d'écoulement du gaz de craquage gazeux, l'eau d'alimentation conduite étant chauffée à des températures d'environ 150° C à environ 300° C.

10. Procédé, destiné à un système de trempe selon la revendication 7 du brevet, **caractérisé en ce que** sur le TLX-D (26), en mode d'alimentation de liquide, l'on conduit l'eau d'alimentation vers le ballon à vapeur (59) par l'intermédiaire de la conduite d'arrivée d'eau d'alimentation (49) montée et d'une tubulure d'eau d'alimentation (50) placée sur le ballon à vapeur.

11. Procédé, destiné à un système de trempe selon la revendication 7 du brevet, **caractérisé en ce que** l'on intègre l'échangeur thermique dual du TLX-D ou TLX-D (26) dans le système de vapeur saturée ou système de refroidissement du système de trempe, de l'eau provenant du ballon à vapeur (59) étant conduite par l'intermédiaire de la conduite descendante (38) du TLX-D, la soupape ouverte de conduite descendante (39) du TLX-D jusqu'à sa distribution vers les tubulures de conduite descendante (40, 41, 42) du TLX-D montées sur le TLX-D.

12. Procédé, destiné à un système de trempe selon la revendication 11 du brevet, **caractérisé en ce que** sur le TLX-D (26), l'on conduit de l'eau provenant du ballon à vapeur (59) par l'intermédiaire de la conduite descendante (38) du TLX-D, du côté de l'enveloppe jusqu'à la tubulure de conduite ascendante (43, 44, 45) du TLX-D, que l'on place à l'opposée des tubulures de conduite descendante (40, 41, 42) du TLX-D, du gaz de craquage circulant à travers le TLX-D (26) n'étant pas sensiblement refroidi, pas au-delà d'un refroidissement de 15 % de la température d'entrée du gaz de craquage, de préférence de moins de 10 %.

13. Procédé, destiné à un système de trempe selon la revendication 12 du brevet, **caractérisé en ce que** sur le TLX-D (26), la température d'entrée de gaz de craquage est proche de 50 °C, se situe de préférence à moins de 30° C, au-dessus de l'ordre de température de la vapeur saturée, **en ce que** du côté de l'eau ou du côté d'enveloppe de l'enveloppe du TLX-D (28) par transition de phase partielle de l'eau, l'on conduit une faible quantité de vapeur produite, moins de 10 t/h de vapeur, de préférence moins de 5 t/h de vapeur, par l'intermédiaire des tubulures de conduite ascendante (43, 44, 45) du TLX-D, par l'intermédiaire de la conduite ascendante (46) du TLX-D, par l'intermédiaire de la soupape ouverte de conduite ascendante (47) du TLX-D et de la tubulure de la conduite ascendante (48) du ballon à vapeur dans le ballon à vapeur (59).

14. Procédé, destiné à un système de trempe selon la revendication 13 du brevet, **caractérisé en ce que** lors de la conception du volume libre ou de l'espace vapeur (61) ou du méplat des déflecteurs (62), l'on prend en compte que le méplat des déflecteurs (62) soit défini d'une faible dimension telle, que d'une part il ne soit donné naissance à aucun courant de dérivation intempestif lors du fonctionnement du TLX-D (26) en tant que préchauffeur de l'eau de dérivation en mode d'alimentation de gaz, et est conçu à une dimension si importante, que d'autre part, lors du fonctionnement du TLX-D (26) en tant qu'évaporateur, la fraction de vapeur provoquée par transition partielle de phase de l'eau en mode d'alimentation de liquide soit complètement évacuée, la hauteur maximale de la section transversale du méplat étant conçue dans un ordre d'environ 5 mm à 40 mm, de préférence de 10 mm à 15 mm.
